# EUROPEAN PATENT APPLICATION

(11) **EP 3 885 441 A1**
(43) Date of publication of application: **29.09.2021**
(21) Application number: 19916825.3
(22) Date of filing: 27.02.2019
(51) Int. Cl.: C12P 13/00, C12P 1/00, C12R 1/125

(54) **BIOLOGICAL SOLID STATE FERMENTATION PROCESS**

(71) Applicant: Fertinagro Biotech, S.L., 44195 Teruel (ES)
(72) Inventor: ATARES REAL, Sergio, 44195 Teruel (ES); ROMERO LOPEZ, Joaquín, 44195 Teruel (ES); SALAET MADORRAN, Ignasi, 44195 Teruel (ES); FERRER GINES, María, 44195 Teruel (ES); CABALLERO MOLADA, Marcos, 44195 Teruel (ES); YANCE CHAVEZ, Tula del Carmen, 44195 Teruel (ES); FUERTES DOÑATE, Carlos, 44195 Teruel (ES)
(74) Representative: González López-Menchero, Álvaro Luis
(86) International application number: PCT/ES2019/070116
(87) International publication number: WO 2020/174101

(57) **Abstract**

The invention provides a solid state biological fermentation process wherein an animal waste feedstock with a high protein nitrogen content is transformed into a final product of high value for various industrial processes, such as the fertiliser, composting industry and the like. The process essentially comprises selecting those microorganisms present in the animal waste feedstock that have the capacity to transform organic nitrogen into protein nitrogen, i.e., into amino acids and peptides, culturing said microorganisms in a suitable medium and adding said microorganisms in the optimal culture medium thereof to the previously sterilised or inactivated feedstock.

## Description

The present invention relates to a solid state biological fermentation process, the process providing the transformation of an animal waste feedstock with a high protein nitrogen content into a final product of high value for various industrial processes, such as the fertiliser, composting industry and the like.

The present invention would fall within the field of Industrial Biotechnology, understood as the field related to technologies to produce goods and services using biological organisms and materials found in nature, modified or not.

Thus, the invention provides a solid state biological fermentation process wherein an animal waste feedstock containing protein nitrogen is transformed, by means of this process, into a product rich in peptides and amino acids, of high value in the aforementioned industries.

Most biotechnological processes are based on microorganisms. The most frequently used organisms are bacteria and yeasts, but other cell cultures can also be used, including tissues or organs. Due to the different implications thereof from the bioprocess point of view, liquid phase and solid phase processes are distinguished.

Particularly, in solid phase processes the diffusion processes of both matter and heat have great importance, and they can become controlling. The designs of these bioreactors are absolutely different from liquid phase bioreactors, wherein transport processes are more intense. They are very common in the food industry, but also in the environmental/energy and health industries (Rendueles, M.; Diaz, M. (2014). "Biotecnología industrial [Industrial biotechnology]". Arbor, 190 (768): a155. doi: http://dx.doi.org/10.3989/arbor.2014.768n4009).

There are many processes that treat solid waste from a biotechnological point of view, specifically the solid phase fermentation processes are present in the uses such as composting, obtaining biogas or obtaining fertilisers or feed.

In this context, international patent application WO2006082264, for example, describes a method wherein waste plant products with a high content of protein nitrogen and from various industries are revalued by means of the transformation thereof, in a two-step process, fermentation and hydrolysis, in a liquid biostimulant product for plants, *Bacillus licheniformis* being used as a microorganism that produces hydrolytic enzymes.

Solid state fermentation (sometimes referred to in the literature as SSF in abbreviated form), is defined as the growth of microorganisms in solid, or semi-solid media, in the absence of free water, the main advantage thereof being the fact that it enables the use of very low-cost substrates, such as agro-industrial waste.

Thus, numerous solid fermentation processes are known wherein plant waste is used as starting materials. See, in this regard, for example, document CN1974782 wherein an animal food product is obtained or the article "Conversion de residuales agroindustriales en productos de valor agregado por fermentación en estado sólido *[Conversion of agro-industrial waste into value-added products by means of solid state fermentation]*" (Bermúdez Savon, Rosa Catalina et al. Conversión de residuales agroindustriales en productos de valor agregado por fermentación en estado sólido. RTQ [online]. 2014, vol.34, n.3 [cited 2019-02-26], pp.263 -274)], wherein the application of solid state fermentation in the biotransformation of lignocellulosic agricultural by-products is described, using white-rot basidiomycete fungi. As an example of this technology, the culturing of the edible mushrooms *Pleurotus spp* on coffee pulp is shown.

The present invention provides a solid state biological fermentation process wherein an industrial animal waste with a high protein nitrogen content is used as feedstock, which is transformed, by means of this process, into a product rich in peptides and amino acids.

In this regard, industrial animal waste has a high protein content and also a high cost for the destruction thereof, therefore, the processes for use thereof are of particular interest. Slaughterhouse waste blood is a by-product that is difficult to revalue, although it contains high amounts of usable proteins. Plasma has traditionally been used as a food additive, especially in meat products (preserves, ready meals, etc.). Methods have also been developed to obtain and use the globin, a major protein of the cellular fraction of the blood, by means of discoloration thereof for use in food. Plasma fractions can further be used (Rendueles, M.; Diaz, M. (2014). "Biotecnología industrial [Industrial biotechnology]". Arbor, 190 (768): a155. doi: http://dx.doi.org/10.3989/arbor.2014.768n4009).

Therefore, the present invention, in addition to providing a final product with high added value, enables this type of animal waste to be used that, otherwise, are difficult to process for reuse thereof.

The process of the invention essentially comprises selecting those microorganisms present in the animal waste feedstock that have the capacity to transform organic nitrogen into protein nitrogen, i.e., into amino acids and peptides, culturing said microorganisms in a suitable medium and adding said microorganisms in the optimal culture medium thereof to the previously sterilised or inactivated feedstock.

The invention is described in more detail below based on an embodiment thereof and with reference to the attached figures, wherein:
Figure 1: Schematic representation of the method of the invention;
Figure 2: Graph showing the results as hydrolysis % in a biological fermentation in a single cycle of the process of the invention;
Figure 3: Graph showing the results as hydrolysis % in a biological fermentation in several cycles of the process of the invention;
Figure 4: Graph showing the results as hydrolysis % in a comparative biological fermentation with type microorganisms.

The solid state biological fermentation process of the present invention is characterised then in that it comprises the following steps:
i) Providing an animal waste feedstock, such as blood meal, meat and bone meal, feathers and the like, as well as combinations of these animal feedstocks;
ii) Carrying out a metagenomic analysis of the microbiota of said feedstock to select those microorganisms with a high capacity to produce specific enzymes capable of hydrolysing proteins, particularly proteases, subtilins, trypsins and pepsins;
iii) Culturing the selected microorganisms in a suitable culture medium;
iv) Sterilising-inactivating the initial feedstock by means of thermal treatment;
v) Adding the culture of the selected microorganisms to the initial inactivated feedstock;
vi) Facilitating the biological fermentation of the culture in the inactivated feedstock by controlling the pH conditions, humidity and temperature.
vii) Analysing the fermentation yield and, if appropriate, repeating steps v) and vi).

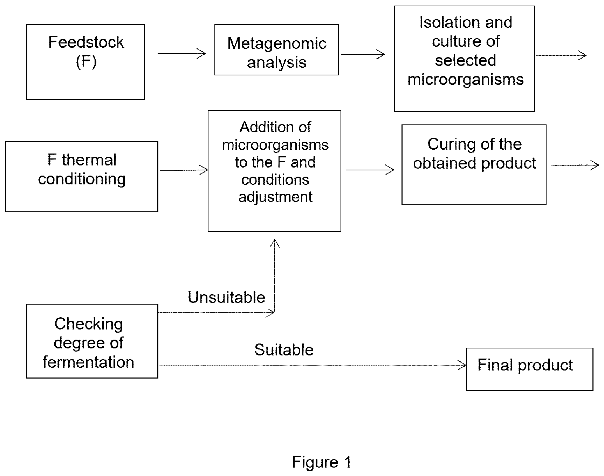

Figure 1 shows a schematic representation of the indicated process.

As mentioned, an animal waste feedstock is provided first, such as blood meal, meat meal, feathers and the like, and combinations thereof.

In this regard and in the context of the present process, blood meal or meat meal is understood as the product that, once dried, accompanies tallow in waste fusion processes. This is a product very rich in nitrogen, phosphorus and calcium and is currently used as an element of animal feed. It may contain high protein content (up to 60%). If it contains more than 4.4% phosphorus, it is called "bone meal". Depending on the type of process used in the fusion, the meal may have a higher protein value (dry fusion process), bone meal is prepared from bones cleared of fat and meat.

In a preferred embodiment, meat meal is used as animal waste feedstock.

The step of metagenomic analysis of the microbiota present in the feedstock is carried out using a metagenomic sequencer in order to study the microbiological interest of the selected feedstock and isolate therefrom those microorganisms with the cited specific functions, using selective culture media wherein said microorganisms are sown.

Thus, out of the possible culture media known, a medium is selected wherein it can be appreciated quickly, with the growth of the microorganisms, the ability thereof to hydrolyse proteins, transforming organic nitrogen into protein nitrogen, i.e., to obtain amino acids.

In a preferred embodiment, this culture medium consists of an agar-calcium-caseinate medium, that facilitates the isolation of these microorganisms when observing halos around the colonies that have said function.

Next, the culture of the selected microorganisms is carried out, the selective design of cultures with pre-enrichment media being necessary. As it is known, the pre-enrichment medium is a solution of nutrient-rich salts, especially with a high contribution of carbon and nitrogen.

In the present invention, the pre-enrichment medium is a mixture with a specific concentration of nutrients for the growth and reproduction of the selected microorganisms to synthesise proteases.

Preferably, the optimal growth conditions for the specific microorganism in this culture medium are a constant temperature between 25 °C and 35 °C, a pH of 5.5 to 8.5 and suitable dissolved oxygen conditions.

In a preferred embodiment of the process of the invention, the selected microorganisms are *Bacillus subtilis,* particularly *Bacillus subtilis* with strain number 9187 and *Bacillus subtilis* with strain number 9265, both deposited in the Spanish Type Culture Collection (CECT).

In this preferred embodiment, both microorganisms were cultured in optimal conditions for the growth thereof using a pre-enrichment medium consisting of a composition of salts including macro- and micro-elements, yeast extract as a nitrogen source, glucose, saccharose, dextrose, maltose as a carbon source and as extra casein input, for example LB (Luria Bertani) medium, and were stabilised with a selective medium of *Bacillus* for the subsequent addition thereof to the feedstock in step v).

Step iv) of the above-described process is preferably carried out at a temperature of 65 °C to 95 °C for at least 4 hours in order to inactivate any microorganisms present in the feedstock.

Preferably, in step v), the selected culture is added to the inactivated feedstock in a proportion of 0.5 to 3.0% by weight, this culture containing between 10⁹ and 10¹⁰ CFU.

The incorporation of this culture to the feedstock further enables the pH to be adjusted in a range of 6.5-8.5 and an optimum humidity of 40% in step vi) of the process, which is preferably carried out at a temperature of 35 °C-55 °C for a period of between 4 and 8 hours.

Once the fermentation is finished, which can take a period of at least 96 hours approximately, in step vii) the fermentation yield is analysed and, in the event of not being that which is desired for the final product, steps v) and vi) are repeated.

### Examples

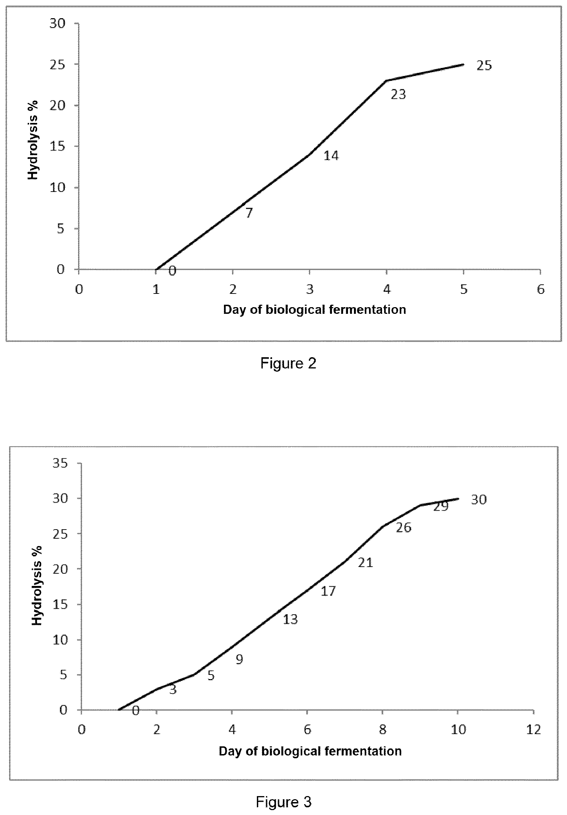

In a laboratory scale test, the process of the invention was carried out with meat meal as starting material and the yield of the process was studied as a function of the degree of hydrolysis, applying a single cycle of steps v) and vi). The results are shown in Figure 2, a hydrolysis percentage of 25% being observed in 5 days of biological fermentation.

The same test was carried out applying a cycle of several additions according to steps v) and vi), with the results shown in Figure 3.

Based on the observation of both figures it is concluded that the maximum level of hydrolysis for this process is around 25-30%, a level that, compared to the solid phase fermentation processes described in the literature with these microorganisms, of a maximum of 20%, is clearly superior.

In the same manner, a comparative test of conventional fermentation was carried out with a *Bacillus sp* microorganism in order to compare the results obtained with the preferred strains of the invention, particularly with *Bacillus subtilis* CECT 9265 and *Bacillus subtilis* 9187. Briefly, in the comparative test, *Bacillus sp* was cultured to obtain a microorganism concentration similar to that used in the test and it was inoculated into non-inactivated feedstock.

The obtained results are shown in Table 4, the fermentation using these microorganisms in the process of the invention proving to have a much higher yield than that obtained using *Bacillus sp.*

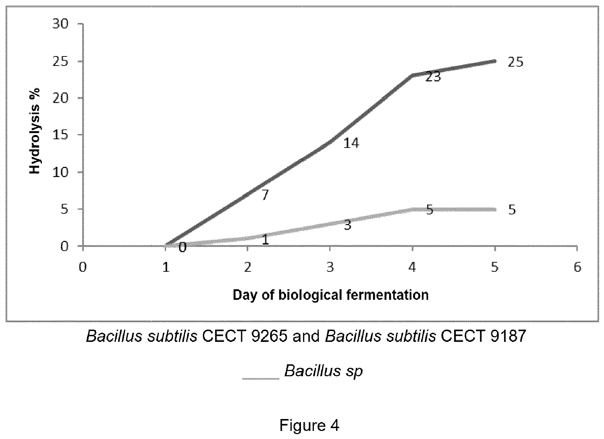

Likewise, an industrial scale test was carried out starting from 100 tons of meat meal, 5 tonnes of culture medium with 20% by weight of microorganisms *Bacillus subtilis* CECT 9265 and *Bacillus subtilis* CECT 9187 at a concentration of 10⁹ CFU and water to achieve a humidity of 40%. The inactivation of the feedstock was carried out for 6 hours at a temperature of 85 °C and the fermentation was carried out for 5 days, a degree of hydrolysis of 23.1% being obtained, as shown in Figure 4.

## Claims

1. A solid state biological fermentation process **characterised in that** it comprises the following steps:
i) Providing an animal waste feedstock;
ii) Carrying out a metagenomic analysis of the microbiota of said feedstock to select those microorganisms with a high capacity to produce specific enzymes capable of hydrolysing proteins, particularly proteases, subtilins, trypsins and pepsins;
iii) Culturing the selected microorganisms in a suitable culture medium;
iv) Sterilising-inactivating the initial feedstock by means of thermal treatment;
v) Adding the culture of the selected microorganisms to the initial inactivated feedstock;
vi) Facilitating the biological fermentation of the culture in the inactivated feedstock by controlling the pH conditions, humidity and temperature;
vii) Analysing the fermentation yield and, if appropriate, repeating steps v) and vi).

2. The solid state biological fermentation process according to claim 1, **characterised in that** the animal waste feedstock is selected from the group consisting of blood meal, meat meal, feathers and the mixtures thereof.

3. The solid state biological fermentation process according to claim 2, **characterised in that** the animal waste feedstock is meat meal.

4. The solid state biological fermentation process according to claim 1, **characterised in that** in step ii) an agar-calcium-caseinate medium is used to facilitate the isolation of microorganisms, when observing halos around the colonies that have the function of hydrolysing proteins.

5. The solid state biological fermentation process according to claim 1, **characterised in that** the culturing of the microorganisms selected from step iii) is carried out at a constant temperature between 25 °C and 35 °C, pH from 5.5 to 8.5.

6. The solid state biological fermentation process according to claim 1, **characterised in that**, as a result of steps ii) and iii) the microorganisms *Bacillus subtilis* are selected and cultured, particularly *Bacillus subtilis* CECT No. 9187 and *Bacillus subtilis* CECT No. 9265.

7. The solid state biological fermentation process according to claim 1, **characterised in that** step iv) is carried out at a temperature of 65 °C to 95 °C for at least 4 hours.

8. The solid state biological fermentation process according to claim 1, **characterised in that**, in step v), the selected culture is added to the inactivated feedstock in a proportion of 0.5 to 3.0% by weight, this culture containing between 10⁹ and 10¹⁰ CFU.

9. The solid state biological fermentation process according to claim 1, **characterised in that** in step vi) the pH is in a range of 6.5-8.5, the humidity is 40% and the temperature is 35 °C-55 °C.
